# FASCICULE DE BREVET EUROPEEN

(11) **EP 2 194 968 B1**
(45) Date de publication et mention de la délivrance du brevet: **19.06.2013**
(21) Numéro de dépôt: 08843692.8
(22) Date de dépôt: 12.08.2008
(51) Int. Cl.: A61K 9/00, A61K 33/14, A61K 35/02

(54) **COMPOSITION THÉRAPEUTIQUE AROMATISÉE À BASE D'ARGILE**
AUF TON BASIERENDE AROMATISIERTE THERAPEUTISCHE ZUSAMMENSETZUNG
CLAY-BASED AROMATISED THERAPEUTIC COMPOSITION

(30) Priorité: 13.08.2007 EP 07291005
(43) Date de publication de la demande: 16.06.2010
(60) Demande divisionnaire: 13002485.4
(73) Titulaire: IPSEN PHARMA, 92100 Boulogne-Billancourt (FR); Firmenich SA, 1211 Geneva 8 (CH)
(72) Inventeur: BARRA, Jérôme, F-74160 Neydens (FR); LE HAZIF, Denis, F-28500 Vernouillet (FR)
(74) Mandataire: Audonnet, Nathalie
(86) Numéro de dépôt international: PCT/FR2008/001185
(87) Numéro de publication internationale: WO 2009/056703

(56) Documents cités:
- EP-A- 0 919 239
- DATABASE WPI Week 200377 Thomson Scientific, London, GB; AN 2003-826431 XP002469755 & KR 2003 050 958 A (SONG W Y) 25 juin 2003 (2003-06-25)
- DATABASE WPI Week 200745 Thomson Scientific, London, GB; AN 2007-468777 XP002469756 & WO 2007/051427 A (HAILISHENG PHARM CO LTD) 10 mai 2007 (2007-05-10)
- DATABASE WPI Week 198641 Thomson Scientific, London, GB; AN 1986-267853 XP002469757 & JP 61 194014 A (KUNIMINE KOGYO KK) 28 août 1986 (1986-08-28)

## Description

La présente demande concerne une composition pharmaceutique aromatisée et contenant une smectite comme principe actif.

Une composition thérapeutique à base de smectite dénommée "diosmectite" existe et est commercialisée sous la marque Smecta^{®}. Mais en raison du goût particulier de l'argile qui peut parfois gêner certains patients et les enfants en particulier, le problème était donc de trouver une nouvelle composition aromatisée et dont le goût reste prédominant par rapport au goût de l'argile. Pour résoudre ce problème, la déposante propose une nouvelle composition pharmaceutique aromatisée, à base de smectite et telle que définie ci-dessous.

La présente invention a donc pour objet une composition thérapeutique aromatisée contenant une argile comme principe actif, et caractérisée en ce que
- l'argile est une smectite dioctaédrique ; et
- l'arôme est encapsulé.

Une composition selon la présente invention peut être utilisée pour la prévention et/ou le traitement de certaines pathologies telles que le traitement symptomatique des douleurs liées aux affections oesogastroduodénales et coliques, les diarrhées aiguës et chroniques, la maladie coeliaque.

Les smectites représentent une famille particulière d'argile dans laquelle on trouve des espèces dioctaédriques telles que montmorillonite et beidellite, et des espèces trioctaédriques telle que hectorite et saponite.

L'argile utilisée selon l'invention est une smectite dioctaédrique. De préférence, la smectite dioctaédrique est une montmorillonite ou une beidellite ou une structure cristallographique intermédiaire entre les deux pôles cristallochimiques : montmorillonite et beidellite. Cette structure cristallographique intermédiaire peut être proche du pôle montmorillonite et même très proche du pôle montmorillonite ; elle peut également être proche du pôle beidellite et même très proche du pôle beidellite. De préférence, une smectite selon l'invention est une montmorillonite ou une structure intermédiaire proche du pôle montmorillonite, et de manière très préférentielle très proche du pôle montmorillonite.

De manière préférentielle également, l'argile utilisée est la smectite dénommée "diosmectite" et commercialisée sous la marque Smecta^{®}.

La composition thérapeutique selon la présente invention comprend "un composé ou une composition aromatisés", appelés "arôme". Le terme "arôme" tel qu'utilisé dans la présente demande couvre les composés ou compositions aromatisés habituellement utilisés dans l'industrie alimentaire, qu'ils soient d'origine naturelle ou synthétique. Il comprend des composés seuls ou des mélanges.

Des exemples spécifiques de tels composés peuvent être trouvés dans la littérature, comme par exemple dans Fenaroli's Handbook of Flavor Ingredients, 1975, CRC Press ; Synthetic Food adjuncts, 1947, M. B. Jacobs, édité par Van Nostrand ; ou Perfume and Flavor Chemicals, 1969, S. Arctander, Montclair, New Jersey (USA).

Ces composés sont bien connus de l'homme du métier dans le domaine des produits de consommation aromatisés ou parfumés, c'est-à-dire des produits de consommation traditionnellement aromatisés dont un parfum ou un arôme ou un goût a été apporté, ou des produits de consommation dont le goût a été modifié.

De manière préférentielle, l'arôme est un liquide hydrophobe, soluble dans les solvants organiques mais seulement très faiblement soluble dans l'eau.

De manière très préférentielle, l'arôme est caractérisé par un paramètre de solubilité de Hildebrand δ inférieur à 30 MPa^{1/2}. L'incompatibilité avec l'eau de la plupart des arômes et parfums peut être en fait exprimée par le biais du paramètre de solubilité de Hildebrand qui est en général inférieur à 25 MPa^{1/2} alors que le même paramètre pour l'eau est de 48 MPa^{1/2} et pour les alcanes de 15-16 MPa^{1/2}. Ce paramètre fournit une échelle de polarité utile corrélé à une densité d'énergie cohésive des molécules. Pour qu'un mélange spontané se produise, la différence de solubilité des molécules à mélanger doit est être maintenue au minimum. Le handbook des paramètres de solubilité (Handbook of Solubility Parameters, A. F. M. Barton, CRC Press, Bocca Raton, 1991) donne une liste de valeurs de δ pour un grand nombre de produits chimiques mais également des méthodes recommandées de contribution de groupes permettant de calculer les valeurs δ pour des structures chimiques complexes.

De préférence également, l'arôme est sous forme liquide, volatile ou labile, avec un log P compris dans la fourchette de -2 à 7, et de manière très préférentielle, de 2 à 6.

De manière préférentielle également, une composition selon la présente invention comprend, en tant qu'arôme, des extraits naturels, des huiles essentielles ou un mélange de ces derniers.

Comme arôme approprié, on peut citer les arômes traditionnels tels que réglisse, fruits exotiques, fruits rouges, les extraits d'agrume tels que citron (vert, jaune), orange, pamplemousse, ou les huiles de mandarine ou café, thé, menthe, cacao, vanille/caramel ou des huiles essentielles d'herbes et de d'épices, ou bien encore des arômes dits "modernes" tels que coca, thé vert, crème brûlée.

De préférence, une composition selon l'invention comprend au moins un arôme encapsulé différent de la vanille.

Les arômes sont de préférence choisis parmi les parfums traditionnels tels que réglisse, fruits exotiques, fruits rouges, les extraits d'agrumes, vanille/caramel/chocolat mais également des parfums dit "modernes" tels coca, thé vert, crème brûlée.

De préférence, l'arôme est choisi parmi les arômes vanille/caramel/chocolat et extraits d'agrumes et de manière très préférentielle, vanille/caramel/chocolat et orange, citron, pamplemousse ou clémentine.

De préférence également, l'arôme est un mélange d'arômes, et de manière très préférentielle un mélange d'arômes vanille et orange.

L'arôme peut être mélangé avec des solvants, des adjuvants, des additifs et/ou d'autres ingrédients, par exemple ceux couramment utilisés dans l'industrie des arômes et/ou de l'alimentaire.

L'arôme selon la présente invention est de préférence encapsulé dans une matrice vitreuse (matrice d'encapsulation) d'hydrate(s) de carbone.

Comme constituant(s) de la matrice d'encapsulation, on peut utiliser tout sucre ou dérivé du sucre qui peut être transformé par des techniques d'extrusion afin de former un solide extrudé sec. Des exemples particuliers de constituants appropriés peuvent être choisis parmi les produits suivants : sucrose, glucose, lactose, levulose, fructose, maltose, ribose, dextrose, isomalt, sorbitol, mannitol, xylitol, lactitol, maltitol, pentatol, arabinose, pentose, xylose, galactose, hydrolysats d'amidon hydrogéné, maltodextrine, Stabilite (nom commercial ; origine : SPI Polyols, USA), agar-agar, carraghénane, d'autres gommes, polydextrose et les dérivés et mélanges des ces derniers.

De préférence, la maltodextrine ou des mélanges de maltodextrine et d'au moins un produit choisi parmi : sucrose, glucose, lactose, levulose, maltose, fructose, isomalt, sorbitol, mannitol, xylitol, lactitol, maltitol et hydrolysats d'amidon hydrogéné sont utilisés. De manière très préférentielle, la matrice est constituée de maltodextrine ou de mélange de maltodextrine et d'au moins un produit choisi parmi : sucrose, maltose, isomalt, maltitol et hydrolysats d'amidon hydrogéné. De manière très préférentielle également, la matrice est constituée de maltodextrine et de sucrose.

De préférence, la maltodextrine a un équivalent-dextrose (DE, Dextrose equivalent) au dessus de 5 et inférieur à 20.

Un émulsifiant tel que la lécithine, et/ou un plastifiant, typiquement de l'eau, peuvent être ajoutés au mélange si nécessaire.

Le produit ou la composition aromatisés selon l'invention, qui sont extrudés, peuvent être préparés par n'importe quelle méthode standard. Par exemple, les procédés décrits dans les brevets US 4,610,890 et US 4,707,367, dont le contenu est incorporé par référence, sont appropriés pour fournir des arômes encapsulés tels qu'utilisés dans la présente invention.

De préférence également, l'arôme est présent à un taux d'au moins 10 % en poids exprimé en matière sèche, par rapport au poids total (exprimé en matière sèche) de la matrice d'encapsulation, et de préférence à un taux compris entre 15 et 35 %.

Une composition selon l'invention comprend de préférence de 70 à 90 % en poids de principe actif, et de manière très préférentielle de à 75 à 85 %, par rapport au poids total de la composition.

De manière préférentielle également, une composition selon l'invention comprend de 0,1 à 3 % en poids d'arôme encapsulé par rapport au poids total de la composition, et très préférentiellement de 0,3 à 2,5 %. De manière très préférentielle également, une composition selon l'invention comprend de 0,5 à 2 % en poids d'arôme encapsulé par rapport au poids total de la composition.

Une composition thérapeutique selon la présente invention peut se présenter sous différentes formes solides comme par exemple des poudres, des granules, des comprimés ou des gélules. Les supports solides appropriés peuvent être, par exemple, le talc, les sucres, le lactose, la dextrine, la gélatine, la cellulose et ses esters.

Dans une composition thérapeutique selon la présente invention, on peut également trouver d'autres additifs tels que des agents colorants, édulcorants, lubrifiants, glidants. Une composition selon la présente invention peut également contenir des minéraux.

Le ou les agents colorants utilisés selon la présente invention peuvent être tout type de colorants habituellement utilisé dans l'alimentaire et/ou la pharmacie. Parmi les agents édulcorants, on peut citer par exemple la saccharine, l'aspartame, la maltodextrine, les monosaccharides tels fructose ou glucose, les disaccharides tels saccharose. Parmi les agents lubrifiants, on peut citer par exemple le talc. Parmi les agents glidants, on peut citer le saccharose.

L'apport en minéraux peut consister par exemple en l'ajout de sels métalliques tels que des sels d'aluminium ou de magnésium tels que l'hydroxyde d'aluminium, le carbonate de magnésium.

Le mode d'administration d'une composition selon l'invention sera choisi, entre autre, en fonction de sa forme galénique et de la pathologie à traiter. De préférence, la composition telle que définie ci-dessus sera administrée par voie orale.

La dose d'administration journalière sera la dose habituelle recommandée pour ce produit. Dans le cas particulier de la smectite dénommée "diosmectite", elle peut être administrée à une dose journalière maximale de 18 g/jour.

A moins qu'ils ne soient définis d'une autre manière, tous les termes techniques et scientifiques utilisés ici ont la même signification que celle couramment comprise par un spécialiste ordinaire du domaine auquel appartient cette invention.

### Partie expérimentale

### Exemple 1 : Préparation d'un arôme (huile) orange encapsulé

Un sirop est préparé à partir de maltodextrin, sucrose, eau et arôme. Le mélange est alors chauffé jusqu'à 123° C pour réduire le taux d'humidité du sirop. Ensuite, l'émulsifiant est mélangé au sirop concentré dans des conditions de cisaillements forts pour former une masse fondue uniforme. La masse fondue est alors extrudée sous une pression de 2x10⁵ Pa à travers une plaque de matrice d'extrusion avec des trous de 0,8 mm de diamètre dans un solvant froid afin de rafraîchir et casser les extrudats.

| Ingrédients | | en gramme (g) | % de matière sèche |
|---|---|---|---|
| | Maltodextrin 18DE | 1505 | 44,55 |
| | Sucrose | 1505 | 44,55 |
| | Huile orange Valence pressée à froid | 350 | 10,36 |
| | Lécithine de soja ¹⁾ | 18 | 0,54 |
| | Eau | 400 | - |
| | Total | | 100,00 |

| | | | |
|---|---|---|---|
| ¹⁾origine : Central Soya, Fort Wayne, Indiana, USA | | | |

### Exemple 2 : Préparation d'un arôme vanille encapsulé

L'exemple 1 est reproduit en utilisant un extrait de vanille (ex Firmenich) à la place de l'arôme orange.

### Exemple 3 : Préparation d'un arôme caramel encapsulé

L'exemple 1 est reproduit en utilisant un arôme caramel (ex Firmenich) à la place de l'arôme orange.

### Exemple 4 : Préparation d'une composition thérapeutique aromatisée

Les compositions ci-dessous sont préparées en mélangeant ensemble, doucement, tous les ingrédients dans les proportions indiquées, jusqu'à ce qu'ils soient dispersés de façon homogène. Dans le tableau ci-dessous, toutes les quantités sont exprimées en mg.

| Ingrédients | Exemple 4a | Exemple 4b | Exemple 4c |
|---|---|---|---|
| Diosmectite | 3000 | 3000 | 3000 |
| Arôme encapsulé orange (arôme préparé selon l'exemple 1) ¹ | - | - | 10 |
| Arôme encapsulé vanille (arôme préparé selon l'exemple 2) ² | 50 | 50 | 50 |
| Arôme encapsulé caramel® (arôme préparé selon l'exemple 3) ³ | 10 | - | - |
| Saccharine soluble + glucose hydraté | 700 | 710 | 700 |
| Eau | 100 | 100 | 100 |

| | | | |
|---|---|---|---|
| ¹ ex Firmenich, ref. 501289 TD 0990B ;² ex Firmenich, ref. 501465 TD1591 ; ³ ex Firmenich, ref. 501403 TD 1094 | | | |

### Exemple 5 : Stabilité d'une composition selon l'invention

L'argile étant de par sa nature un produit stable, la stabilité d'une composition selon l'invention est mesurée à la fois au niveau des caractéristiques organoleptiques de l'arôme et de son inertie vis-à-vis de l'argile.

La stabilité des caractéristiques organoleptiques de l'arôme est constatée par des testeurs (panel de 7 experts) sur la base de nombreux critères (sucré, fruité, acide, amer,...) pour une durée d'au moins 6 mois.

Par ailleurs il est constaté que l'arôme ne présente aucune dégradation en présence de l'argile pour cette même période.

## Revendications

1. Composition thérapeutique aromatisée contenant une argile comme principe actif, et **caractérisée en ce que**
- l'argile est une smectite dioctaédrique ; et
- l'arôme est encapsulé.

2. Composition selon la revendication 1, **caractérisée en ce que** la smectite dioctaédrique est une montmorillonite ou une beidellite ou une structure cristallographique intermédiaire entre les deux pôles cristallochimiques : montmorillonite et beidellite.

3. Composition selon l'une des revendications précédentes, **caractérisée en ce que** la smectite dioctaédrique est une montmorillonite ou une structure cristallographique intermédiaire proche du pôle montmorillonite.

4. Composition selon l'une des revendications précédentes, caractérisée en ce la smectite dioctaédrique est une montmorillonite ou une structure cristallographique intermédiaire très proche du pôle montmorillonite.

5. Composition selon l'une des revendications précédentes, **caractérisée en ce que** la smectite dioctaédrique est la smectite dénommée "diosmectite".

6. Composition selon l'une des revendications précédentes, **caractérisée en ce que** l'arôme est un liquide hydrophobe.

7. Composition selon l'une des revendications précédentes, **caractérisée en ce que** l'arôme présente un paramètre de solubilité de Hildebrand inférieur à 30 MPa^{1/2}.

8. Composition selon l'une des revendications 1 à 5, **caractérisée en ce que** l'arôme est sous forme liquide avec un log P compris dans la fourchette de -2 à 7.

9. Composition selon la revendication précédente, **caractérisée en ce que** l'arôme présente un log P compris dans la fourchette de 2 à 6.

10. Composition selon l'une des revendications précédentes, **caractérisée en ce que** l'arôme est un extrait naturel, une huile essentielle ou un mélange de ces derniers.

11. Composition selon l'une des revendications précédentes, **caractérisée en ce qu'**elle comprend au moins un arôme encapsulé différent de la vanille.

12. Composition selon l'une des revendications précédentes, **caractérisée en ce que** l'arôme est choisi parmi les parfums suivants : réglisse, fruits exotiques, fruits rouges, extraits d'agrumes, vanille, caramel, chocolat, coca, thé vert, crème brûlée.

13. Composition selon l'une des revendications précédentes, **caractérisée en ce que** l'arôme est choisi parmi les parfums suivants : vanille, caramel, chocolat et extraits d'agrumes.

14. Composition selon l'une des revendications précédentes, **caractérisée en ce que** l'arôme est choisi parmi les parfums suivants : vanille, caramel, chocolat, orange, citron, pamplemousse et clémentine.

15. Composition selon l'une des revendications précédentes, **caractérisée en ce que** l'arôme est un mélange d'arômes.

16. Composition selon la revendication précédente, **caractérisée en ce que** l'arôme est un mélange d'arômes vanille et orange.

17. Composition selon l'une des revendications précédentes, **caractérisée en ce que** l'arôme est encapsulé dans une matrice vitreuse d'hydrate(s) de carbone.

18. Composition selon la revendication précédente, **caractérisée en ce que** la matrice est constituée de maltodextrine ou de mélange de maltodextrine et d'au moins un produit choisi parmi : sucrose, glucose, lactose, levulose, maltose, fructose, isomalt, sorbitol, mannitol, xylitol, lactitol, maltitol et hydrolysats d'amidon hydrogéné.

19. Composition selon l'une des revendications 17 à 18, **caractérisée en ce que** la matrice est constituée de maltodextrine ou de mélange de maltodextrine et d'au moins un produit choisi parmi : sucrose, maltose, isomalt, maltitol et hydrolysats d'amidon hydrogéné.

20. Composition selon l'une des revendications 17 à 19, **caractérisée en ce que** la matrice est constituée de maltodextrine et de sucrose.

21. Composition selon l'une des revendications 18 à 20, **caractérisée en ce que** la maltodextrine a un équivalent-dextrose au dessus de 5 et inférieur à 20.

22. Composition selon l'une des revendications précédentes, **caractérisée en ce que** l'arôme est présent à un taux d'au moins 10 % en poids exprimé en matière sèche, par rapport au poids total (exprimé en matière sèche) de la matrice d'encapsulation.

23. Composition selon l'une des revendications précédentes, **caractérisée en ce que** l'arôme est présent à un taux compris entre 15 et 35 % en poids exprimé en matière sèche, par rapport au poids total (exprimé en matière sèche) de la matrice d'encapsulation.

24. Composition selon l'une des revendications précédentes, **caractérisée en ce qu'**elle comprend de 70 à 90 % en poids de principe actif.

25. Composition selon l'une des revendications précédentes, **caractérisée en ce qu'**elle comprend de 75 à 85 % en poids de principe actif.

26. Composition selon l'une des revendications précédentes, **caractérisée en ce qu'**elle comprend de 0,1 à 3 % en poids d'arôme encapsulé.

27. Composition selon l'une des revendications précédentes, **caractérisée en ce qu'**elle comprend de 0,3 à 2,5 % en poids d'arôme encapsulé.

28. Composition selon l'une des revendications précédentes, **caractérisée en ce qu'**elle comprend de 0,5 à 2 % en poids d'arôme encapsulé.

## Patentansprüche

1. Aromatisierte therapeutische Zusammensetzung, die einen Ton als Wirkstoff enthält und **dadurch gekennzeichnet ist, dass**
- der Ton ein dioktaedrischer Smektit ist und
- der Aromastoff eingekapselt ist.

2. Zusammensetzung nach Anspruch 1, **dadurch gekennzeichnet, dass** der dioktaedrische Smektit ein Montmorillonit oder ein Beidellit oder eine kristallografische Zwischenstruktur zwischen den zwei kristallochemischen Polen Montmorillonit und Beidellit ist.

3. Zusammensetzung nach einem der vorangehenden Ansprüche, **dadurch gekennzeichnet, dass** der dioktaedrische Smektit ein Montmorillonit oder eine kristallografische Zwischenstruktur ist, die dem Montmorillonit-Pol nahe kommt.

4. Zusammensetzung nach einem der vorangehenden Ansprüche, **dadurch gekennzeichnet, dass** der dioktaedrische Smektit ein Montmorillonit oder eine kristallografische Zwischenstruktur ist, die dem Montmorillonit-Pol sehr nahe kommt.

5. Zusammensetzung nach einem der vorangehenden Ansprüche, **dadurch gekennzeichnet, dass** der dioktaedrische Smektit der Smektit ist, der "Diosmektit" genannt wird.

6. Zusammensetzung nach einem der vorangehenden Ansprüche, **dadurch gekennzeichnet, dass** der Aromastoff eine hydrophobe Flüssigkeit ist.

7. Zusammensetzung nach einem der vorangehenden Ansprüche, **dadurch gekennzeichnet, dass** der Aromastoff einen Löslichkeitsparameter nach Hildebrand von unter 30 MPa^{1/2} aufweist.

8. Zusammensetzung nach einem der Ansprüche 1 bis 5, **dadurch gekennzeichnet, dass** der Aromastoff in flüssiger Form mit einem log P, der im Bereich von -2 bis 7 liegt, vorliegt.

9. Zusammensetzung nach dem vorangehenden Anspruch, **dadurch gekennzeichnet, dass** der Aromastoff einen log P, der im Bereich von 2 bis 6 liegt, aufweist.

10. Zusammensetzung nach einem der vorangehenden Ansprüche, **dadurch gekennzeichnet, dass** der Aromastoff ein natürlicher Extrakt, ein etherisches Öl oder ein Gemisch dieser letztgenannten ist.

11. Zusammensetzung nach einem der vorangehenden Ansprüche, **dadurch gekennzeichnet, dass** sie wenigstens einen eingekapselten Aromastoff umfasst, der von Vanille verschieden ist.

12. Zusammensetzung nach einem der vorangehenden Ansprüche, **dadurch gekennzeichnet, dass** der Aromastoff aus den folgenden Aromen ausgewählt ist: Lakritze, exotische Früchte, rote Früchte, Zitrusfruchtextrakte, Vanille, Karamell, Schokolade, Kakao, grüner Tee, Crème brûlée.

13. Zusammensetzung nach einem der vorangehenden Ansprüche, **dadurch gekennzeichnet, dass** der Aromastoff unter den folgenden Aromen ausgewählt ist: Vanille, Karamell, Schokolade und Zitrusfruchtextrakte.

14. Zusammensetzung nach einem der vorangehenden Ansprüche, **dadurch gekennzeichnet, dass** der Aromastoff aus den folgenden Aromen ausgewählt ist: Vanille, Karamell, Schokolade, Orange, Zitrone, Pampelmuse und Klementine.

15. Zusammensetzung nach einem der vorangehenden Ansprüche, **dadurch gekennzeichnet, dass** der Aromastoff ein Gemisch von Aromastoffen ist.

16. Zusammensetzung nach dem vorangehenden Anspruch, **dadurch gekennzeichnet, dass** der Aromastoff ein Gemisch aus den Aromastoffen Vanille und Orange ist.

17. Zusammensetzung nach einem der vorangehenden Ansprüche, **dadurch gekennzeichnet, dass** der Aromastoff in einer glasartigen Matrix aus Kohlenhydrat(en) eingekapselt ist.

18. Zusammensetzung nach dem vorangehenden Anspruch, **dadurch gekennzeichnet, dass** die Matrix aus Maltodextrin oder einem Gemisch aus Maltodextrin und wenigstens einem Produkt, ausgewählt aus: Saccharose, Glucose, Lactose, Levulose, Maltose, Fructose, Isomalt, Sorbit, Mannit, Xylit, Lactit, Maltit und Hydrolysaten hydrierter Stärke, besteht.

19. Zusammensetzung nach einem der Ansprüche 17 bis 18, **dadurch gekennzeichnet, dass** die Matrix aus Maltodextrin oder einem Gemisch aus Maltodextrin und wenigstens einem Produkt, ausgewählt aus: Saccharose, Maltose, Isomalt, Maltit und Hydrolysaten hydrierter Stärke, besteht.

20. Zusammensetzung nach einem der Ansprüche 17 bis 19, **dadurch gekennzeichnet, dass** die Matrix aus Maltodextrin und Saccharose besteht.

21. Zusammensetzung nach einem der Ansprüche 18 bis 20, **dadurch gekennzeichnet, dass** das Maltodextrin ein Dextrose-Äquivalent von über 5 und unter 20 hat.

22. Zusammensetzung nach einem der vorangehenden Ansprüche, **dadurch gekennzeichnet, dass** der Aromastoff in einem Verhältnisanteil von wenigstens 10 Gew.-%, ausgedrückt als Trockensubstanz, bezogen auf das Gesamtgewicht (ausgedrückt als Trockensubstanz) der Einkapselungsmatrix vorliegt.

23. Zusammensetzung nach einem der vorangehenden Ansprüche, **dadurch gekennzeichnet, dass** der Aromastoff in einem Verhältnisanteil zwischen 15 und 35 Gew.-%, ausgedrückt als Trockensubstanz, bezogen auf das Gesamtgewicht (ausgedrückt als Trockensubstanz) der Einkapselungsmatrix vorliegt.

24. Zusammensetzung nach einem der vorangehenden Ansprüche, **dadurch gekennzeichnet, dass** sie 70 bis 90 Gew.-% Wirkstoff umfasst.

25. Zusammensetzung nach einem der vorangehenden Ansprüche, **dadurch gekennzeichnet, dass** sie 75 bis 85 Gew.-% Wirkstoff umfasst.

26. Zusammensetzung nach einem der vorangehenden Ansprüche, **dadurch gekennzeichnet, dass** sie 0,1 bis 3 Gew.-% eingekapselten Aromastoff umfasst.

27. Zusammensetzung nach einem der vorangehenden Ansprüche, **dadurch gekennzeichnet, dass** sie 0,3 bis 2,5 Gew.-% eingekapselten Aromastoff umfasst.

28. Zusammensetzung nach einem der vorangehenden Ansprüche, **dadurch gekennzeichnet, dass** sie 0,5 bis 2 Gew.-% eingekapselten Aromastoff umfasst.

## Claims

1. Flavoured therapeutic composition containing a clay as active ingredient, and **characterized in that**
- the clay is a dioctahedral smectite; and
- the flavour is encapsulated.

2. Composition according to claim 1, **characterized in that** the dioctahedral smectite is a montmorillonite or a beidellite or an intermediate crystallographic structure between the two crystal-chemical poles: montmorillonite and beidellite.

3. Composition according to one of the previous claims, **characterized in that** the dioctahedral smectite is a montmorillonite or an intermediate crystallographic structure close to the montmorillonite pole.

4. Composition according to one of the previous claims, **characterized in that** the dioctahedral smectite is a montmorillonite or an intermediate crystallographic structure very close to the montmorillonite pole.

5. Composition according to one of the previous claims, **characterized in that** the dioctahedral smectite is the smectite known as "diosmectite".

6. Composition according to one of the previous claims, **characterized in that** the flavour is a hydrophobic liquid.

7. Composition according to one of the previous claims, **characterized in that** the flavour has a Hildebrand solubility parameter less than 30 MPa^{1/2}.

8. Composition according to one of claims 1 to 5, **characterized in that** the flavour is in liquid form with a log P comprised within the range -2 to 7.

9. Composition according to the previous claim, **characterized in that** the flavour has a log P comprised within the range 2 to 6.

10. Composition according to one of the previous claims, **characterized in that** the flavour is a natural extract, an essential oil or a mixture thereof.

11. Composition according to one of the previous claims, **characterized in that** it comprises at least one encapsulated flavour different from vanilla.

12. Composition according to one of the previous claims, **characterized in that** the flavour is chosen from the following flavours: liquorice, exotic fruits, red fruits, extracts of citrus fruits, vanilla, caramel, chocolate, coca, green tea, crème brêlée (caramel custard).

13. Composition according to one of the previous claims, **characterized in that** the flavour is chosen from the following flavours: vanilla, caramel, chocolate and extracts of citrus fruits.

14. Composition according to one of the previous claims, **characterized in that** the flavour is chosen from the following flavours: vanilla, caramel, chocolate, orange, lemon, grapefruit and clementine.

15. Composition according to one of the previous claims, **characterized in that** the flavour is a mixture of flavours.

16. Composition according to the previous claim, **characterized in that** the flavour is a mixture of vanilla and orange flavours.

17. Composition according to one of the previous claims, **characterized in that** the flavour is encapsulated in a glassy matrix of carbohydrates.

18. Composition according to the previous claim, **characterized in that** the matrix is constituted by maltodextrin or a mixture of maltodextrin and at least one product chosen from: sucrose, glucose, lactose, levulose, maltose, fructose, isomalt, sorbitol, mannitol, xylitol, lactitol, maltitol and hydrogenated starch hydrolysates.

19. Composition according to one of claims 17 to 18, **characterized in that** the matrix is constituted by maltodextrin or a mixture of maltodextrin and at least one product chosen from: sucrose, maltose, isomalt, maltitol and hydrogenated starch hydrolysate.

20. Composition according to one of claims 17 to 19, **characterized in that** the matrix is constituted by maltodextrin and sucrose.

21. Composition according to one of claims 18 to 20, **characterized in that** the maltodextrin has a dextrose equivalent greater than 5 and less than 20.

22. Composition according to one of the previous claims, **characterized in that** the flavour is present in a proportion of at least 10% by weight expressed as dry matter, with respect to the total weight (expressed as dry matter) of the encapsulation matrix.

23. Composition according to one of the previous claims, **characterized in that** the flavour is present in a proportion comprised between 15 and 35% by weight expressed as dry matter, with respect to the total weight (expressed as dry matter) of the encapsulation matrix.

24. Composition according to one of the previous claims, **characterized in that** it comprises from 70 to 90% by weight of active ingredient.

25. Composition according to one of the previous claims, **characterized in that** it comprises from 75 to 85% by weight of active ingredient.

26. Composition according to one of the previous claims, **characterized in that** it comprises from 0.1 to 3% by weight of encapsulated flavour.

27. Composition according to one of the previous claims, **characterized in that** it comprises from 0.3 to 2.5% by weight of encapsulated flavour.

28. Composition according to one of the previous claims, **characterized in that** it comprises from 0.5 to 2% by weight of encapsulated flavour.
